Europäisches Patentamt

European Patent Office

Office européen des brevets

(19)

(11) Publication number: **0 245 206**
**A1**

# (12) EUROPEAN PATENT APPLICATION

(21) Application number: **87810274.8**

(22) Date of filing: **30.04.87**

(51) Int. Cl.⁴: **C 12 Q 1/68,** G 01 N 21/75,
G 01 N 33/543, G 01 N 21/64,
G 01 N 33/545

(30) Priority: **05.05.86 EP 86810201**

(43) Date of publication of application: **11.11.87**
**Bulletin 87/46**

(84) Designated Contracting States: **BE CH DE ES FR GB IT LI
NL SE**

(71) Applicant: **BATTELLE MEMORIAL INSTITUTE, 7 route de
Drize, CH-1227 Carouge/Genève (CH)**

(72) Inventor: **Sutherland, Ranald Macdonald, 8 sis Rue du
Roi-Victor-Amé§App. 21, CH-1227 Carouge (CH)**
Inventor: **Bromley, Peter, 26, Ch. du Pont de Ville,
CH-1224 Chene-Bougeries (CH)**
Inventor: **Gentile, Bernard, 6b, Chemin du Saux,
CH-1111 Tolochenaz (CH)**

(74) Representative: **Dousse, Blasco et al, 7, route de Drize,
CH-1227 Carouge/Genève (CH)**

(54) Analytical method for detecting and measuring specifically sequenced nucleic acid.

(57) A waveguide coated with single-stranded probe nucleic acids and carrying an internally reflected wave signal is contacted with an analyte solution containing denatured test DNA or RNA and fluorescent marker dye. Analyte nucleic acid with sequences homologous to that of the probe polynucleotide will hybridize therewith with concomitant binding of the fluorescent dye to the resultant duplex structures. Fluorescence resulting from the interaction of the wave signal at the waveguide/analyte interface with the signal generating centers created within the space probed by the evanescent component of the wave signal is detected and provides useful information on said sequences homologous to that of the probe nucleic acids.

EP 0 245 206 A1

## ANALYTICAL METHOD FOR DETECTING AND MEASURING SPECIFICALLY SEQUENCED NUCLEIC ACIDS

The present invention relates to a method for determining nucleic acids containing a particular sequence of bases. The determination of such nucleic acids, for instance in DNA or RNA present in biological samples, is of great significance for biomedical and genetic investigations.

There is currently a clinical need to detect specific nucleotide sequences in biological samples, e.g. body fluids, tissue samples such as chorionic villus samples, and swabs. The results from such analyses can be used to identify and/or detect disorders ranging from genetic abnormalities and cancer susceptibility, to infectious diseases of bacterial, viral and fungal origin. As yet, however, the methods used for such nucleotide detection are not readily applicable to a routine environment, and cannot be carried out without specialised staff and laboratory facilities. This is due to the complexity of the procedures, the requirement for hazardous materials (e.g. radioisotopes), and the lengthy incubation times required to achieve a result.

Conventionally, the desired nucleic acid sequence is detected by incubation with the complementary nucleotide which is pre-labelled for detection following the reaction. This complementary sequence (or probe) can be readily synthesised using state of the art technology.

The power of nucleic acid probe technology stems from the specificity of the detection procedure due both to the uniqueness of nucleic acid sequences and the availability of stringent hybridization conditions that allow the discrimination between perfect and imperfect sequence matching. It is possible to discriminate between two sequences present in DNA that differ by a single nucleotide in sequence using suitably sized probes and specific hybridization conditions. This allows the detection of the presence of single point mutations in the test sequence, and where such mutations may be correlated with disease, such as specific cancers and others, these procedures will find application in the diagnosis of such diseases. Secondly, the development of automated synthesis systems of specific oligonucleotide sequences has greatly facilitated the construction of probes of base sequence derived from the extensive and rapidly growing nucleic acid data bases which are currently available.

Conventionally, detection of specific sequences of DNA for example is a three stage procedure (P. SZABO et al., Trends in Biochemical Sciences (1982), pp. 425-427). Firstly, the hybridization coupling between the sample, generally attached to a solid substrate, and a probe-DNA is effected. Secondly, the non-specifically bound probe-DNA is washed away and, thirdly, the specifically bound DNA is determined. Historically the probe-DNA has been radiolabelled by incorporation of $^{32}P$ as an intrinsic label in the sugar-phosphate "back-bone", or by $^{125}I$ as an extrinsic label incorporated into the aromatic structure of the bases. Thus by autoradiography or isotope counting techniques the current detection systems have achieved very high sensitivity, of the order of one genome per cell (Hasse E.T. et al. (1982). Virology 119: 339-410). Isotopic labelling to quantitate the extent of binding is a thus well established technique in immunoassays. However, in view of the hazards involved in working with radioactive compounds, great efforts have been made in the last ten years to avoid the use of isotopes in immunoassay and, instead, to use enzyme, fluorescent or chemiluminescent labels. With the large current experience gained in immunoassays, the search for non-isotopic labels for nucleic acids has started. However, because of the rather harsh conditions of hybridization tests, it is not possible to use enzymes labels since they would denature under current hybridization conditions.

Hence, a number of developments aimed at overcoming the inherent undesirability of radioisotopic labels and nucleic acid probes and also the extended reaction times and specialised equipment required to visualise the $^{32}P$-labelled DNA have been undertaken. Davidson and co-workers have covalently linked biotin to RNA via cytochrome C or polyamine bridges. After hybridation with the sample, the probe is determined by reaction with avidin immobilized on methacrylate spheres. Thus, with the spheres acting as labels the hybridization sites can be detected by electron microscopy (J.E. MANNING et al., Chromosoma 53 (1975), 107-117; A. SOJA et al., Nucleic Acid Research 5 (1978), 385-401). This procedure has been successfully applied in chromosome mapping procedures. In an attempt to design a system with more general applicability, Langer et al (Proceedings of the National Academy of Sciences, USA 78 (1981), 6633-6637) synthetized a number of nucleotide analogues containing biotin covalently attached to the pyrimidine or purine ring. Biotin labelled derivatives of uridine triphosphate (UTP) and dUTP were produced by a complex series of chemical reactions. These analogues were successfully incorporated into DNA by reaction in vitro with a range of DNA polymerases. The modified DNA molecule (probe) was then used to bind either avidin or anti-biotin antibodies. Here both the avidin and antibody signal generators were colorimetrically ascertained using peroxidase-chromogen systems. Although successfully demonstrated, this approach has a number of drawbacks: Firstly there is the technically demanding preparation of relatively unstable nucleoside-triphosphate-biotin analogues. Secondly enzymic incorporation of such analogues into DNA is difficult to control, inefficient and results generally in a maximum of one to two biotin molecules per probe-DNA molecule. Finally, the affinity of biotin antibodies for biotin (at best $10^8$ to $10^{10}$) is considerably less than that of avidin. A further development of this use of nucleotide analogues was demonstrated by Vincent et al., (Nucleic Acid Research (1982) 10, 6787-6796) who, following a similar approach to Langer et al., constructed a dinitro-phenol (DNP) nucleotide analogue. This analogue was successfully incorporated into the DNA at an approximate ratio of one to two molecules of DNP per probe molecule. Detection of the probe was carried out by reaction with an anti-DNP antibody, followed by detection of specifically bound antibody by reaction with an anti-species antibody-peroxidase conjugate. However, it was found that the system was not sufficiently sensitive when compared to isotopic techniques. One major reason was due to a proportion of the antigen (DNP) molecules being bound within the hybridization complex, thus being unavailable for reaction with antibody.

Recently, improved techniques for detecting DNA based on using fluorescence signal generators and linking probe-DNA to substrates were developed. For instance, FR-A-2.480.943 (WANG et al; ABBOTT laboratories) discloses a new class of dyestuffs which fluoresce strongly when in contact with nucleic acids, particularly double stranded DNA. In the absence of the target nucleic acids, there is no fluorescence in aqueous solution and relatively little fluorescence with single stranded polynucleotides (mRNA). Measurements are performed on bulk solutions by means of a fluorescence microscope. For instance concentrations 1-100 nM of DNA can be measured using aqueous 3-$\gamma$-dimethylaminopropyl-2-p-dimethylaminostyrylbenzothiazolium iodide (2 mg/ml). The fluorescence is linearly proportional to the amount of DNA in the sample.

Document EP-A-57.553 (BIRNBOIM; Atomic Energy of Canada) teaches the use of fluorescent dyes which interact with duplex DNA providing fluorescence, but no fluorescence with single stranded DNA. This technique enables to measure the extent of DNA denaturation due to heat, pH, chemicals, radiations or other denaturing conditions. Suitable dyes are ethidium bromide; 4',6-diamino-2-phenylindole dihydrochloride; mithramicin, Hoechst 33258 and others. Fluorescence is measured on bulk solutions.

US-A-4,423,153 (D.F. RANNEY et al) concerns a specific DNA-fluorochrome adduct the formation of which imparts fluorescence enhancement in direct proportion to the total double stranded DNA present in a sample. The preferred fluorescence enhancer is mithramicin binding specifically to duplex DNA. Fluorescence is measured in bulk with a fluorimeter. Several convenient commercial fluorimeters are mentioned specifically.

Document US-A-4,257,774 (RICHARDSON et al; Meloy lab.) discloses the direct binding of fluorescent intercalators to DNA, e.g. ethidium salts, daunomycin, mepacrine and acridine orange as well as 4',6-diamidino-2-phenylindole to quantitate the DNA. Fluorescence polarization is used in tests for the determination of non-fluorescent DNA binders which normally compete with the binding of said dyes, thus leading to corresponding decrease in fluorescence.

In addition, probes immobilized on supports have been disclosed. For instance, Document EP-A-131830 (DATTAGUPTA et al; Molecular Diagnostics) discloses labelling nucleic acids for detection in hybridization for the determination of DNA containing specific base-pair sequences. The labelled nucleic acid comprises at least one single stranded base sequence substantially complementary to or homologous with the sequence to be detected. The probe can be immobilized on a solid support such as nitrocellulose, modified nylon and fluorinated hydrocarbons in the form of filters or sheets. For labelling, enzymes or biotin-avidin systems are disclosed. Similarly, EP-A-130,523 (DATTAGUPTA et al; Molecular Diagnostics) discloses the immobilization of nucleic acid probes on solid substrates. Substrates with reactive groupings such as carboxyl, amino and hydroxy, e.g. cotton, paper, carboxymethyl cellulose, etc.. are suitable.

Nucleic acids are linked to the substrates using photochemically reactive nucleic acid-binding ligands, e.g. intercalators like furocoumarin, angelicin, psoralen and aminophenanthridium halides or non-intercalators such as neotropsin, distamicin, Hoechts 33258 and bis-benzimidazole. Coupling of ligands and substrates is effected by bridging compounds such as CNBr, dialdehydes and di-glycidyl ethers.

Detection of substrate immobilized fluorescent generators was also investigated. Thus, EP-A-70687 (M.J. HELLER; AMOCO CORP.) deals with reactant immobilization in DNA hybridation. For instance, an immobilized single-stranded polynucleotide is hybridized with a light-emitting labelled complementary polynucleotide reagent. After separation of the unhybridized reagent from the immobilized sample, the light label is excited and the light response is detected. The quoted immobilization supports are glass beads, polyacrylamide, agarose, Sephadex, cellulose etc. The light labels can be chemiluminescent, bioluminescent, phosphorescent or fluorescent. For detection, commercially available detectors can be used, e.g. photomultiplier tubes.

Also, EP-A-117.777 (THANH THUONG NGUYEN et al, CNRS) discloses tailor-made oligonucleotides covalently attached to an intercalator group such as acridine, furocoumarin and ellipticin derivatives to be used as nucleic acid sequences recognition systems. Measurements based on bulk or gel fluorescence are recited.

Despite the indubitable achievements of the prior art, it was felt that further improvements were desirable, especially with regard to more rapid and sensitive tests and avoidance of many of the skilled manipulations associated with current techniques.

This was accomplished according to the method of the present invention which mainly relies on using an optical waveguide as the probe immobilization support, the fluorescent markers used to label the polynucleotide probe hybridization products interacting with the wave energy travelling in said waveguide to provide a fluorescent signal which is detected and measured at an output of said waveguide. This method is briefly summarized in annexed claim 1. In this claim, determination is intended to cover detection, identification, quantification, concentration measurements and the like to be performed on an analyte.

Specifically, the nucleic acid probe is linked by usual or novel means to an optically transparent support which functions as a light guide, or waveguide. The single-stranded sample nucleic acid is allowed to react with the probe in the presence of intercalant fluorescent dyes. Such dyes insert themselves between the strands of double stranded nucleic acids (i.e. they intercalate). Following intercalation there is an enhancement of the dye fluorescence quantum yield, an increase in fluorescence half-life and a red-shift of the fluorescence emission wavelength all of which can be monitored optically. The probe is immobilized on the waveguide surface either before or after hybridization but only the fluorescent centers of the duplex bound to the waveguide may provide the fluorescent signal used for the determination.

It is noteworthy that by conventional transmission photometry it would not be possible to simultaneously differentiate between hybridisation of molecules affixed to the support surface and hybridisation reactions occuring in the solution. However, by internally reflecting the fluorescence
excitation light within the waveguide support, i.e. discriminating that
part of the optical signal arising from interaction of the evanescent
vector with the complex at the waweguide surface analyte from other optical
changes occuring within the solution, the hybridisation reaction at the
waveguide surface can be measured. Using this approach in an embodiment of
the present invention, kinetic measurement of nucleic acid hybridisation
reactions were carried out, without the necessity to pre-prepare labelled
probes, without the necessity to allow the reaction to go to completion (as
monitoring is in a real time) and without the necessity to separate the
other materials from the system prior to measurement (as only the surface
reaction is monitored).

Further detail will now be provided with reference to the annexed
drawing.

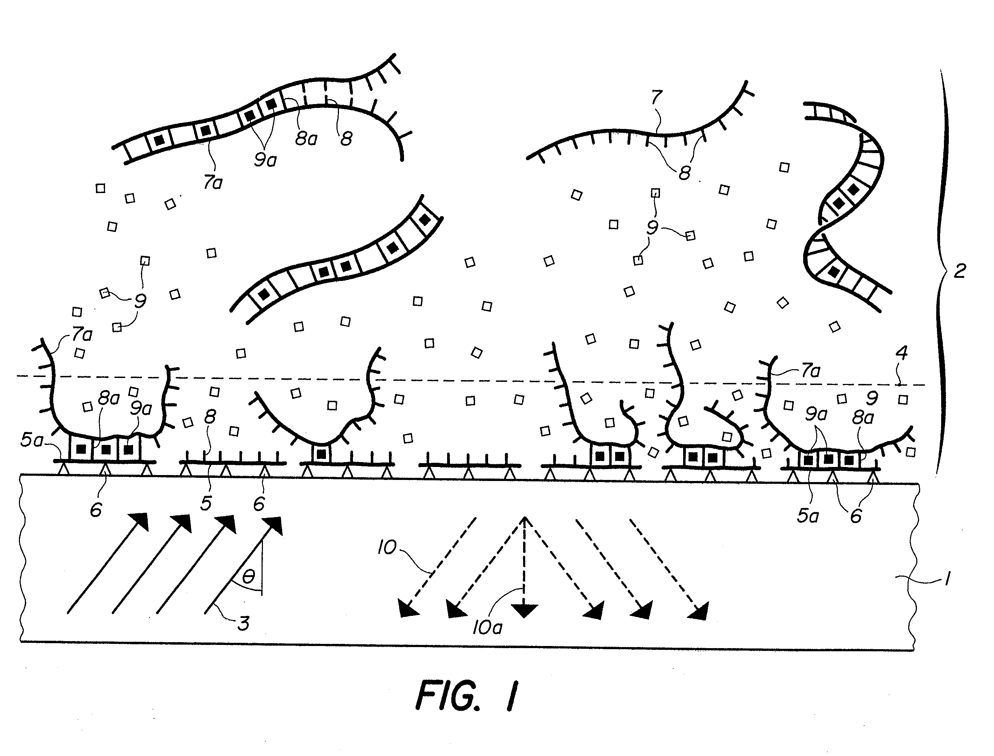

In this drawing, fig. 1 represents schematically a hybridization
reaction occuring with polynucleotides featuring complementary base sequences, some of which (the probe) are linked to the surface of a support.

Fig. 2 represents schematically one preferred mode of linking the
probe to the support's surface.

Fig. 3 illustrates schematically the mode of propagation of a beam of
light by internal reflection in a waveguide of refractive index nl greater
than than n2 of the surrounding medium.

Fig. 4 i.e. fig. 4A, 4B, 4C and 4D illustrate schematically embodiment
devices for illuminating a waveguide to be contacted with an analyte containing species which generate an optical signal at the interface with the
waveguide and for detecting and collecting said signal.

Fig. 5 (A & B) illustrate curves representative of the measurements
done with the devices of fig. 4.

Fig. 6 represents another curve like that of Fig. 5.

Fig. 7 represents schematically a hollow cylindrical embodiment of a
waveguide.

Fig. 8 is a diagram illustrating different embodiments of the method
of the invention.

Fig. 1 shows schematically a waveguide 1 in contact with an analyte
solution 2 containing single-stranded nucleic acid chains 7 and intercalating dye molecules 9. The waveguide 1 is coated with oligonucleotide probes
5 immobilized on the waveguide surface by physical or chemical linkers 6.

Nucleotide bases covalently bound to the polynucleotide back-bone are numbered 8. Some of the nucleic acid sample chains 7 have a base sequence homologous to that of the probe 5 and they hybridize therewith to form base pairs 5a-7a. Bond formation between the strands is indicated by numeral 8a. Other hybrids also form within the solution itself as shown in the drawing. Once the duplex structures have formed, the dye molecules 9 intercalate between the strands (9a) and become fluorescent. Normally, one dye molecule intercalates every five base pairs for DNA and every ten pairs for RNA.(J.B. LEPECQ et al., J. Mol. Biol. $\underline{27}$ (1967), 87-106). Excitation light 3 travels through the waveguide by multiple or single internal reflection at an angle $\theta$ larger than the critical angle $\theta_c$. This excitation light interacts at the vicinity of the interphase boundary by its evanescent vector component. The depth of penetration of this light component (a fraction of a wavelength) is adjusted to give the maximum strength electromagnetic field corresponding to interaction with maximum efficiency with the fluorescent centers of the bound complex; actually, one approach to vary this penetration is by changing the angle $\theta$ as disclosed in USP 4,608,344. Thus, improved sensitivity for surface reactions in thin films is when $\theta$ is selected from values very close to $\theta_c$. Another useful reference in this field is N.J. HARRICK, Internal Reflection Spectroscopy, Wyley Interscience, New-York (1967). The fluorescence resulting from this interaction is then mostly reinjected into the waveguide and can be collected and monitored at an output thereof by usual means. Alternatively or in combination, the fluorescence emitted sidewise, e.g. normally (10a) to the waveguide surface can also be collected and measured in some cases, for instance when the waveguide consists of a wall of a cuvette holding the reactants solution and the fluorescence 10a is emitted through the wall toward the outside. One of the key features in the present method is that the signal originating from the fluorescent centers bound to the waveguide surface can be distinguished from that occurring in the bulk of the solution. Therefore the background effect of side reactions occurring in the analyte solution independently of the probe bound to the waveguide can be efficiently cancelled. Thus, in the present method, only double stranded material which is formed at the waveguide surface (i.e. with the probe polynucleotide) is detected since the excitation light only penetrates a fraction of a wavelength beyond the solid/liquid interface. Actually, the fluorescence response is emitted in all directions (10,10a) but has a preferred angular probability close to the incident light reflection criti-

cal angle $\theta_c$.

Another key factor for success is the immobilisation of the probe nucleic acid to the surface of the waveguide either before or after hybridization. There are many technologies available for immobilising biological macromolecules such as nucleic acids to solid supports. These, or modifications thereof, are equally applicable to this invention. However with an optically transparent waveguide certain limitations are imposed where the immobilisation technique should not disadvantageously affect the optical characteristics of the surface. Two basic techniques for immobilising biological compounds to surfaces are physical adsoption and covalent chemical attachment. For reviews see WB Jakoby and M. Wilcheck, (1974). Affinity Techniques, Methods in Enzymology, Vol. XXXIV, Academic Press, NY; Mosbach, K (1976). Immobilised Enzymes, Methods in Enzymology, Vol. XLlV, Academic Press, NY; Scouten, WH (1981). Affinity Chromatography, Wiley Interscience, NY; Meinkoth, J and Wahl, G (1984). Anal. Biochem. 138; 267-284. These techniques have advantages and disadvantages within the current application. Physical adsorption to the waveguide surface may result in a significant proportion of the probe molecules being unavailable for reaction as the nucleotide bases are attached directly to the waveguide surface. Similarly, with covalent linkage of the probe most of the state-of-the art methods rely on covalent reaction between functional groups on the bases and a reactive grouping previously placed on the solid phase. The resultant probe will be attached on multiple-sites with many bases unavailable for hybridization. The ideal system would attach the probe to the surface with all the relevant bases available for reaction with the sample nucleic acid, and the majority of the probe should be immobilised within the penetration depth of the excitation light to give maximum signal after reaction with the sample nucleic acid.

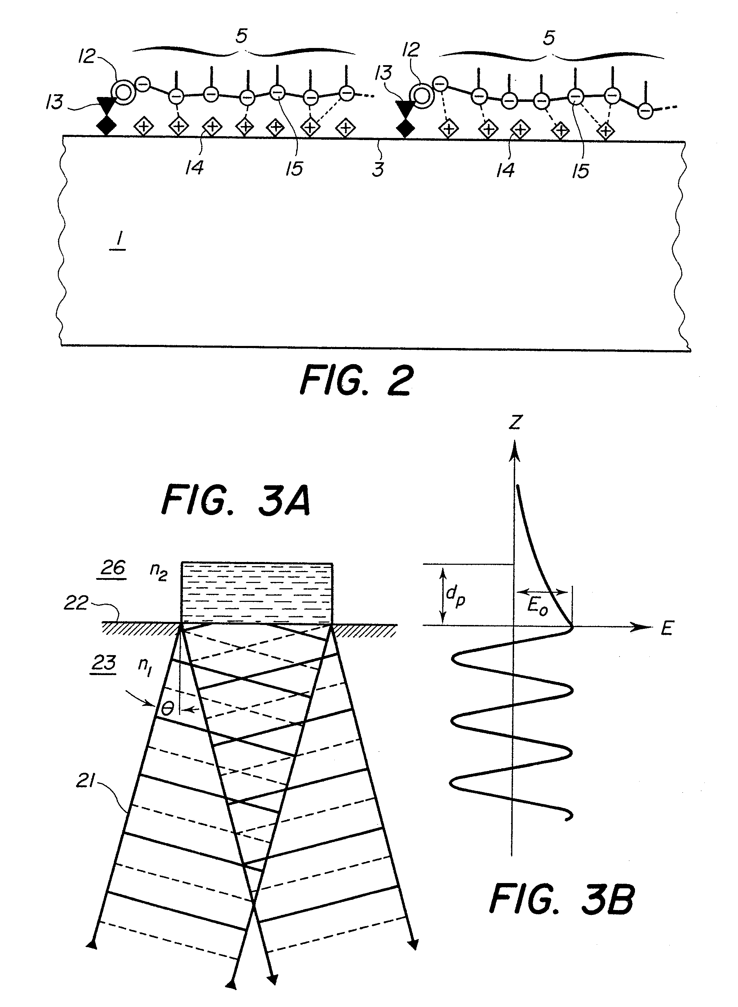

Waveguides having probes attached according to the above consideration have been made available in the present invention. This is illustrated on fig. 2. Here, the terminal nucleotide 12 of the probe 5 is modified (e.g. by its phosphate end) to allow covalent attachment of one end of the probe to an active grouping 13 on the waveguide surface 3. For instance, this grouping 13 can be the $NH_2$ of an amino silane which, after reaction with the probe, leads to a phosphamidate bond. Alternatively, a urethane-phosphamido link of formule $-CONH-(CH_2)_n-NH-P$ is also possible from the reaction of, for instance, an isocyanato-silane and an alkylene diamine. Also on the waveguide surface are molecules 14 deposited with a positive charge facing into the solution 2. The positively charged molecules 14 attract the negatively charged nucleoside phosphate groups 15 in the probe 5. The ratio of groups 13 to positively charged sites is adjusted to allow optimal immobilisation of the probe although keeping the bases 8 free for hybridization, i.e. the equivalent amount of 13 being available will correspond approximately to the molality of the probe whereas the amount of positive centers will depend roughly on its average length (the average number of bases in the segments). The resultant complex of probe:positively charged molecules can give a probe molecule wholly or partially immobilised parallel to the waveguide surface with the relevant bases 8 available for reaction with sample nucleic acids. One embodiment of this system will be detailed in the experimental section. The unexpected advantage of this approach is that by concentrating the DNA probes at such a surface in such a configuration, a rapid sensitive detection method for hybridisation assays is found. Also unexpected is that once formed, this probe:surface molecule complex resists displacement by chemical or competitive binding from other nucleic acid molecules. The positively charged centers on the waveguide can be, for instance, quaternary amino groups. Such groups can be obtained, for example, by quaternizing a dialkylamino end group of a dialkyl-amino-alkyl-amine attached to the waveguide surface, by a quaternizing agent, e.g. an alkyl halide. An example is treating the waveguide surface with trihydroxy- γ -amino propyl silane and quaternizing with methyl bromide.

There is no particular limitation in the choice of the waveguides usable in this invention and most types presently known in the art are usable, including transparent plates, rods, optical fibers and the like. Many of these are disclosed in numerous publications also describing the required illuminating sources, photodetectors and optical configurations for handling the probes and performing corresponding tests. Fluorescence detection analysis and quantification of the signals obtained according to the present method can also be enhanced using photon counting devices (photomultipliers). The differentiation of free from intercalated dye can be made by combining the techniques of this invention with other known fluorescence techniques such as fluorescence polarization and/or time-resolved fluorescence. Other techniques also useful in the present context are Fluorescence Raman Scattering, Fluorescence Correlation Spectroscopy and Fluorescence Fluctuation Spectroscopy. Significant references in this field are "Modern Fluorescence Spectroscopy (198...) Ed. WEBRY, Heydn, New-York; WO-83011230; USP-3,975,084; 3,436,159; 4,447,548; 3,999,855;

3,604,977.

Although the present method mainly relies on measuring a fluorescence signal at an output of a waveguide, i.e. at one or both ends thereof or sidewise thereto, it is not limited to this type of interaction. Indeed, a reaction of nucleic acids at the interface of a waveguide can also be detected by other optical phenomena such as signal absorption, scattering and the like, particularly as the nucleotide bases have strong optical absorption at some specific frequencies. Therefore excitation by radiations in the UV, visible and IR ranges are possible.

Further, although the cited prior art mainly deals with standard waveguides such as prisms, plates, fibers made of glass, quartz or appropriate plastics there is the possibility, in the invention, to enhance the optical interactions at the interface by a number of techniques including the use of very thin waveguides called optical cavities which are effectively very thin (one or more) dielectric layers (of the order of 1-10 microns) deposited on another dielectric (see the aforementioned reference by Harrick, pp. 147-177 and J. DELAY et al, Eur. J. Biochem 12 (1970), 133; V.A.R. HUSS et al., System. Appl. Microbiology 4 (1983), 184-192). Hollow waveguides, i.e. cylindrical or capillary waveguides in which the analyte is circulated are also possible.

Another potential method for enhancing signal from such an interface is to add a thin metal layer (of the order of 500-600 Angströms) between the immobilised nucleic acid and the waveguide. This technique, with the appropriate choice of optical conditions (e.g. metal type, thickness of layer, angle of incident light, polarisation of incident light beam, wavelength of incident light beam) can generate an electromagnetic field in the optically rarer medium, i.e. the sample, up to eighty-fold stronger than without the metal film, (for references , see H. Raether, (1977). Surface Plasmon Oscillations and Their Applications in Physics of Thin Films, Advances in Research and Development, Eds, G. Haas, and MH Francombe, Academic Press, NY. pp. 145-261; H. Raether (1980). Excitation of Plasmons and Interband Transition by Electrons. Springer-Verlag, FRG; B. Liedberg, C. Nylander and I. Lundstrom, (1983). Sens. Actuators 4,299-304). This technique called Surface Plasmon Resonance, can be used as a sensitive detector of a change in the thickness of the layer at the interface. It may also be a method of enhancing the fluorescence intensity at the surface due to the increased intensity of the excitation light magnetic field. Although the above publications detail the principles of internal reflection spectroscopic techniques, for the sake of clarity and continuity of this invention, a brief description will be given as follows with reference to fig. 3.

When a light beam 21 irradiates the interface 22 between two transparent media ($n_1 > n_2$ as shown in fig. 3A, striking from the medium of higher refractive index $n_1$ 23, total internal reflection occurs (Harrick, 1967) when the angle of reflection $\theta$ is larger than the critical angle $\theta_c$, the value of which is given by:

$$\theta_c = \sin^{-1}(n_2/n_1) \tag{1}$$

In this case the evanescent wave penetrates a distance ($d_p$) of the order of a fraction of a wavelength, beyond the reflecting surface into the rarer medium 26. According to Maxwell's equations, a standing sinusoidal wave, perpendicular to the reflecting surface, is established in the denser medium (Fig. 3B). Although there is no net flow of energy into a non-absorbing, rarer medium, there is an evanescent field in that medium. Because of continuity conditions of the field vectors the electric field amplitude (E) is largest at the surface interface ($E_o$) and decays exponentially with distance (Z) from the surface according to:

$$E = E_o \cdot \exp(-Z/d_p)$$

The depth of penetration ($d_p$), defined as the distance required for the electric field amplitude to fall to $\exp(-1)$ of its value at the surface, is given by:

$$d_p = \frac{\lambda/n_1}{2\pi \left[\sin^2\theta - (n_2/n_1)^2\right]^{1/2}}$$

This quantity decreases with increasing $\theta$ and increases with closer index matching (i.e. as $n_2/n_1 \to 1$). Also, because, $d_p$ is proportional to wavelength, it is greater at longer wavelengths.

Thus, by an appropriate choice of the refractive index $n_1$ of the internal reflection element, of the incident angle, and of the wavelength, one can select a $d_p$ to promote optical interaction mainly with compounds close to or affixed at the interface and minimally with bulk solution.

The depth of penetration is one of four factors which determine the attenuation caused by absorbing films in internal reflection.

The other factors are the polarization dependent electric field intensity at the reflecting interface, the sampling area which increases with increasing $\theta$ and matching of the refractive index of the denser medium to that of the rarer medium which in turn controls the strength of the optical coupling. The appropriate quantity which takes account of all these factors is the effective thickness, $d_e$. It represents the actual thickness of film that would be required to obtain the same absorption in a transmission experiment.

In order to enhance sensitivity, multiple reflection elements are often used. The number of reflections (N) is a function of the length (L), thickness (T) of the waveguide and angle of incidence ($\theta$).

$$N = L/T \cdot \cot \theta \qquad (4)$$

The longer and thinner the waveguide, the larger N is and the more frequently the evanescent wave interacts with the surface layer of antibody-antigen complexes. If for one reflection the reflectivity (R) is:

$$R = 1 - \alpha \cdot d_e \qquad (5)$$

where $\alpha$ is the absorption coefficient and $d_e$ is the effective thickness of a weackly absorbing layer, after N reflections the reflection loss is:

$$R^N = 1 - N \cdot \alpha \cdot d_e, \qquad (6)$$

i.e. it is increased by a factor of N.

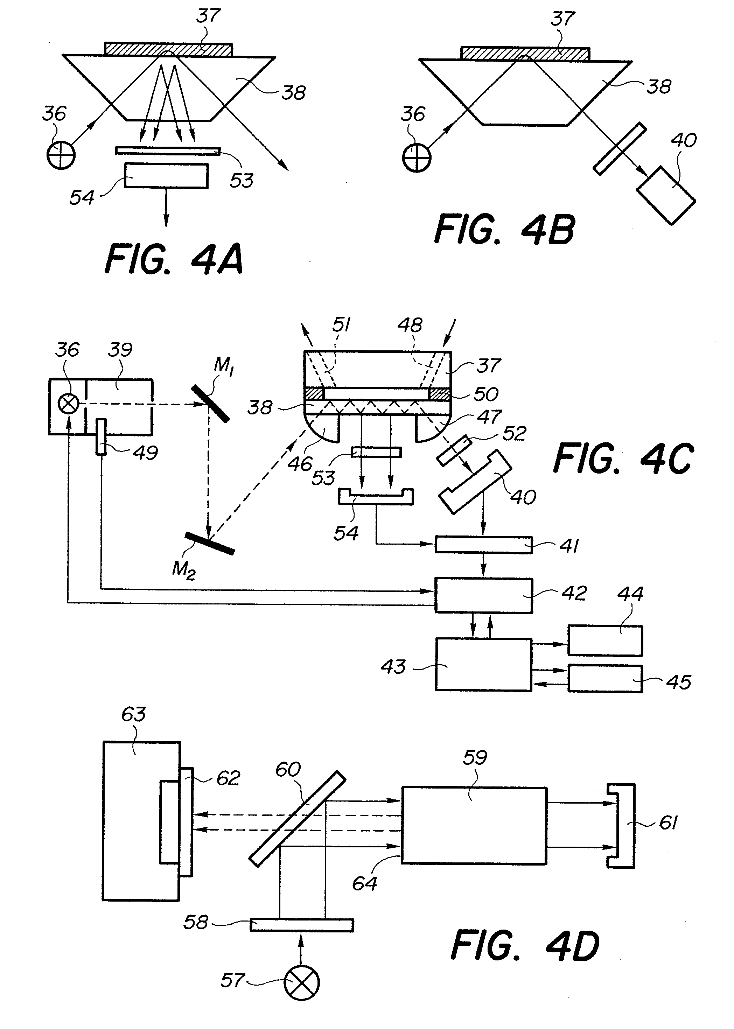

Thus, the evanescent wave can be advantageously used in the present invention to monitor surface reactions by a variety of optical techniques, one of which is detailed below with reference to figs 4A, B and C for illustration, although this is not limiting.

The apparatus used is schematically represented on fig. 4C which shows as a block diagram the major components; these components comprise a monochromator 39, a light source 36, a flow cell 37 with waveguide 38, a filter 52 and electronics with data-acquisition and processing microcomputer including a photomultiplier detector 40, a preamplifier 41, a microprocessor light source control system 42, a micro-computer 43, a printer 44, and a memory (e.g. floppy disc) 45.

To collect the fluorescence at a waveguide/liquid interface alternate signal collection techniques can be employed (fig. 4A and 4B). Fluorescence emitted at an interface can be detected either conventionally where the detector 54 is placed at right angles to the interface (fig. 4A), and/or, as detector 40, in-line with the primary light beam (fig. 4B). Considering the very small solid angle of emission in in-line detection compared with the emission angle of right-angle geometry detection, the former would not seem to be very efficient. However, there is an enhancement effect, and theory predicts that for a fused silica waveguide with water as the $n_2$ medium, the in-line fluorescence intensity can be 50 times higher than the fluorescence emitted at right-angles to the waveguide. This effect, that fluorescence is tunnelled back into the waveguide is verified both theoretically and experimentally (e.g. C.K. Carniglia et al., I. Opt. Soc. Amer. 62 (1972); 479-485).

In a first step, an incident plane wave generates an evanescent wave which excites molecules near the surface with a local distribution proportional to the evanescent electric field intensity (see equation 2). After a characteristic excited-state lifetime, these molecules emit fluorescent radiation with a local distribution in the vicinity of the surface very similar to the exciting intensity distribution described by equation 2 i.e. that of an evanescent wave, but at the fluorescent wavelength. The question of what happens to the fluorescent evanescent wave can be answered by applying the principle of optical reciprocity, which states that this light is coupled back into the waveguide as a plane wave in the same way as the primary process when a plane wave generates an evanescent wave. Theory shows that the fluorescent intensity emission peaks at the critical angle of total internal reflection so that it can be internally reflected. To increase sensitivity, the internal reflected elements can be structured to collect fluorescent light from several reflections and guide it to the detector.

This is especially advantageous when an optical fibre is used as an internal reflection element, as fluorescent light emitted at right-angles from an elongated fibre cannot be easily collected onto a detector. In-line detection also avoids measurement of fluorescence through the bulk of the sample solution surrounding the fibre, which otherwise can give significant interference, depending on the fluorescent dye used.

The light source 36 in the present embodiment was a Xenon flash lamp (E.G. & G. Salem, MA, USA) and the monochromator was equipped with a concave holographic grating (JOBIN-YVON, Paris) to permit a resolution of 5 nm. The flash lamp operation was controlled by microcomputer 42. To inject the samples through an input 48 to the cell 37 a programmable automatic pipette (Microlab-P; Hamilton Bonaduz AG, Bonaduz, Switzerland) was preferably used. The optical component further included two mirrors $M_1$ and $M_2$ and two prisms 46 and 47. A photomultiplier tube of the detector 40 (R928; Hamamatsu, Tokyo, Japan) placed at the waveguide output monitored the change in light intensity directly. Alternatively or simultaneously, a detector 54 with filter 53 provided a signal from the right-angle fluorescence emission (see fig. 4A). Signals from the photomultiplier tubes were amplified (41), integrated during the flash time (42) and converted by a standard 12-bit analog/digital converter (not shown) into digital format. The in-house developed microcomputer 42 performed fast signal averaging, and all data were adjusted for variation in flash lamp intensity by reference to a photodiode 49 placed in the monochromator. The signals were transmitted to a microcomputer 43, preferably an APPLE II model, for display and storage.

The analytical cell or cuvette illustrated on fig. 4C is based on a microscope slide waveguide system. The illustrated system shows the flow cell 37 whose bottom is actually the microscope slide 38. Tightness is ensured by a gasket 50; the slides 38 were placed in direct optical contact by use of index matching oil with two quarter-round silica prisms 46 and 47, preferably from Heraeus. The index matching oil, thereby removed the requirement for specially polished, optically flat waveguide faces. The prisms were designed to allow easy adjustement of the angle of incident light θ (see fig. 3A) and to avoid contact of light with the sealing gasket 50.

The flow cell, machined from aluminium alloy, met the criterion of allowing rapid, bubble-free laminar flow along the light path. Its design also ensured rapid and accurate demounting and repositioning. We chose an aluminium alloy, although other metals are also suitable, e.g. brass, because of its good thermal conductivity, relative lack of reactivity with saline solution, and low optical reflectivity after being anodized matt black to avoid stray light effects. The gasket 50 was 0.5 mm thick medical grade silicone rubber and water tight under a constant sealing pressure of 2 $kg/cm^2$. Including input 48 and exit 51 ports the total cell volume was 1.8 ml, the volume directly above the waveguide was 0.66 ml (53 x 25 x 0.5) and the volume above the light path was 0.29 ml (36 x 16 x 0,5 mm). The afore described apparatus is operated as follows:

In a first step, waveguide plate 38 is coated with probe DNA using the technique described in connection with fig. 2 and detailed hereafter, then the coated waveguide forming the bottom of the analytical cuvette 37 is installed and after turning on the energizing and detecting elements, an analyte solution (containing nucleic acid fragments and a dye is added to the cell whereby hybridization between complementary polynucleotide strands occurs. The dye molecules intercalate between the duplex strands and will provide fluorescence upon excitation. Fluorescence generated at the waveguide interface is therefore emitted at the output of the wave guide and collected by detectors 40 or emitted at right angle to the waveguide and collected by detector 54 after filtering out the remaining excitation component with filter 52 or 53, respectively. The corresponding signal provided by detector 40 is then processed by the remaining components 41-45 of the apparatus to furnish the desired results. The rate of increasing fluorescence is a measure of the hybridization rate and therefore depends on the concentration in the sample of DNA complementary to the probe and its affinity thereto. Fixed time and end point measurements can also be performed to provide similar or related results. Standard tests can be run to calibrate the equipment with known samples of complementary DNA and used thereafter as turnplates to determine unknown samples. Or course, this equipment is also usable in the embodiment where the probe attaches to the waveguide after hybridizing with the polynucleotide to be determined, the only main difference is that the reactants are allowed to react before the mixture is introduced into the cell.

Fig. 4D illustrates an alternative apparatus to detect and monitor the fluorescent light which is tunnelled back through the waveguide and exits therefrom on the input side. The working components of this embodiment are about the same as in the first embodiment and include a source 57, a filter 58, a waveguide 59, a reference detector 61, a fluorescence emission filter 62 and a signal detector 63. It additionally includes a beam splitter 60 which reflects the incident wavelength from the source toward the waveguide.

Here, excitation light generated by source 57 and filtered by filter 58 is reflected onto the light input face 64 of the waveguide 57 via the optical beam splitter 60. Fluorescence light generated at the interface of the waveguide and the analyte solution (not shown) is tunnelled back into the waveguide and exits from face 64. By correct choice of the optical performance of the beam splitter 60, a large proportion of this fluorescent signal is passed directly through the beam splitter to be detected after filtering (62) by detector 63.

The advantage of this design over that illustrated by fig. 4C is that the exciting light is not directly incident on the sample detector 63, thus minimizing background signal levels. The appropriate beam splitter can be a single quartz slide or a more complex dichroic laminate. The latter is preferred as it selectively reflects the excitation light into the waveguide 59 and transmits the fluorescent light to the signal detector 63.

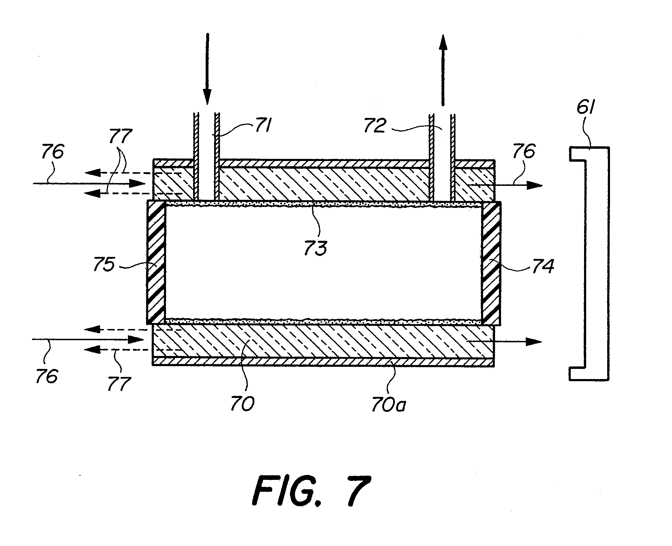

In a variant of the embodiment of fig. 4D, the plain waweguide 59 can be replaced by a hollow waveguide 70 (see fig. 7) in which the liquid to be tested is circulated by input 71 and output type 72. This hollow waveguide with cladding 70a can be a capillary tubing of which the internal area can be very large relative to its internal volume which arrangement provides a high density of interaction sites between the evanescent wave signal and the fluorescent centers of the hybridized material represented conventionally by coating 73. The position of input 71 and output ducts 72 is purely optionnal. In fig. 7 the ends of the tube, which actually acts as a test cuvette, are stoppered by plugs 74 and 75; obviously any other means to hold the liquid in the tube and prevent it from leaking at the ends is valid.

The operation of this waveguide variant is about the same as that illustrated on fig. 4D. The input light shown by arrows 76, propagates through the walls 70 of the waveguide and the evanescent wave component interacts with material 73 and generates a fluorescent signal schematized by the dotted arrows 77. This fluorescence is picked-up by a detector like detector 63 of fig. 4D. For the rest, the set up and operation is exactly like the embodiment of fig. 4D.

The advantage of the tubular waveguide is that it allows circulation of a relatively large volume of solution (held in a reservoir, not represented on the drawing, for instance) or recirculation of the same sample to ensure maximum uptake of all analyte molecules by the probe coated on the inside wall of the tube.

Therefore, even in case the nucleic acid to be detected by hybridization is very diluted, it will progressively attach to the probe as circulation progresses and overall sensitivity will be strongly enhanced.

The dyes used as intercalators have been characterized and investigated and their use in measuring double stranded nucleic acids is state-of-the art. Lists of appropriate dyes are given in EPA 84107624.3 and FR-A-8107928. Of particular interest in this application are the phenanthridine dyes (e.g. ethidium bromide and the ethidium bromide homodimer) and acridine dyes. A review of some of the salient optical and structural properties is given in RH Sarma, (1980), Nucleic Acid Geometry and Dynamics, Pergamon Press, London. Other key references are J-B LePecq and C. Paoletti, (1967). J. Mol. Biol. 27,87-106; J-B LePecq, M. LeBret, J. Barket, and B. Roques, (1975). Proc. Natl. Acad. Sci. (USA). 72, 2915-2919; J. Olmsted and DR Kearns, (1977). Biochem. 16, 3647-3654., M. LeBret and O. Chalvet (1977). J. Mol. Struct. 37, 299-319.; AR. Morgan, DH. Evans, JS Lee and D. Pulleybank, (1979). Nucleic Acids Res. 7, 571-594.; J. Markovits, B-P Roques and J-B LePecq, (1979). Anal. Biochem. 94, 259-264. The important factors relating to these dyes are the changes in spectral characteristics following intercalation of the dye between double stranded nucleic acids. Following intercalation, there is an enhancement of fluorescent lifetime, the fluorescent quantum yield is increased and there is a red shift in the fluorescence emission wavelength. By appropriate choice of the optical system, fluorescence from intercalated dye can be readily differentiated from non-intercalated dye e.g. by choosing the appropriate emission filter. The fluorescent signal is directly proportional to the concentration of double stranded nucleic acid (where the dye is in excess concentration). This is the basis of US patent 4,423,153, a method for detecting the total cellular DNA.

Such dyes have been shown to react with double stranded DNA from many species (e.g. calf thymus DNA, mouse phage T4, E. coli, P. vulgaris, M. lysodeikticus, human DNA, different RNAs, and homopolynucleotides such as polydA:polydT, polydC:polydG (from above references)) and are therefore universally applicable.

The interaction of certain dyes (e.g. Acridine orange) with the double stranded nucleic acid can be inhibited by other intercalators (e.g. Actinomycin D). This is the basis of a test system for detecting compounds that interact with nucleic acids described in US patent 4,257,774.

As the nucleic acid has to be in a double stranded form for intercalation to occur, breakage of this double strand, such as caused by low radiation doses or certain chemicals, can result in a lowered fluorescent signal when compared to control samples not exposed to injurious materials. This is the basis of EPA 82300376.01 as a method for the detection of damaged DNA.

None of these methods can be applied to the detection of specific sequences of nucleotide in a mixture of many nucleic acid molecules, the key aim of the present invention.

The use of photochemicaly activated intercalating compounds for synthesising labelled probes has been advocated in EPA 84107624.3. Here the photochemically active intercalator is linked to the nucleotide and either the dye fluorescence is used to detect the probe after hybridisation or the intercalator is used as a "bridge" to link other materials to be detected as labels e.g. enzymes, avidin-biotin, antibodies. However this approach is directed at conventional hybridisation assays systems and not related to waveguides, non-separation/non-washing/kinetic hybridisation assays and the prior art above technique requires pre-labelling of the probe prior to reaction, not an in situ labelling as in the present method.

lt should be remarked that it is possible to form double-stranded DNA at an interface such as disclosed here, and thereafter label the double stranded DNA with an intercalator which will cross-link the strands. This will allow the immobilized material to be worked without loss of specific signal but with a further increase in sensitivity due to minimal background signals. This may be necessary for applications requiring very high sensitivity. Also a reaction sequence may be envisaged whereby more than one probe is used. For example a type of sandwich hybridization assay (M. RADOKI et al., Gene 21 (1983), 77-85) whereby the sample DNA is reacted with the immobilized probe and a second (or more) fluorescently labelled probe (s) in solution. The resultant complex can be measured at the interface using the technique of the present invention. Advantages of such a system are that it allows use of a "generic" solid-phase probe which can fit a number of selected applications while the specific probe can be added in solution, thus precluding the need to manufacture a range of solid-phase probe-waveguides.

Therefore the present invention is different compared to the prior art and has a number of unique characteristics which makes it very useful for carrying out analyses for clinical diagnostic purposes, and which do not exist in any other current system. These characteristics are specifically: the rapid detection of defined nucleic acid sequences in the kinetic measurement mode; the technical ease of a kit/instrumental system based on this invention due to the lack of requirement to pre-label reagents; no need to wash the solid phase and detection in situ of the hybridised probe; the sensitivity due to the enhanced fluorescence of the intercalated dye and to using internal reflection detection techniques; the specificity due to the nucleic acid probe and using internal reflection to detect only the thin surface film and no other extraneous materials.

For in-the-field uses (e.g. clinical laboratories, agricultural testing laboratories, food technology, raw material quality control, etc.) a kit system is provided by the present invention. Such kit includes the following components: a sampling device (syringe, swab, etc..); a sample preparation device to provide deproteinized solutions of single stranded nucleic acids; then the kit also includes a nucleic acid hybridization system according to the method of this invention. Depending on the embodiment contemplated, the system is a single low-throughput device or a large multi-analyte, multi-sample complete analytical outfit, manual or automatic. In both cases the waveguide is part of a cheap disposable cuvette (e.g. moulded plastic) which has the probe nucleic acid attached on part of the inside surface thereof. All reactions take place within the cuvette and optical measurements involve the coated portions of the cuvette. For the more sophisticated embodiment, it is appropriate to have the probes linked to a reusable device whereby the analytical unit is of the flow-cell type (see fig.4C) with the waveguide as an integral part. In the case of DNA a series of solutions are passed through the cell to allow measurement of base-line signal, specific binding signal after injection of sample and dye, then disruption of the double-stranded DNA and reequilibration of the system ready for accepting the next sample. Disruption of the duplex DNA can be achieved by usual chemical or physical means. The simplest technique to denature duplex DNA is to heat the sample at a temperature which depends on relative base-pair concentration, length of the hybridized molecule and the "goodness-of-fit" of the two annealed strands. There exists thus a mean denaturation temperature (Tm) at which the two strands separate. Not only can this Tm value of temperature be used to separate strands when getting ready for a second sample, but a temperature control device incorporating the features of the present invention have a number of advantages.

The relation between base-pair sequence and Tm is reasonably accurately defined by the equation:

$$Tm = 0.41 \ (G+C) + 13.3 \log X + 69.37$$

where G and C are the equivalent % concentrations of guanine and cytosine, respectively and X is the ionic strength of the buffer. This formula may have to be modified in the presence of certain polymers, e.g. polyethylene glycol, due to exclusion properties of such polymers (see M. GILLIS et al, European Journal of Biochemistry 12 (1970), 143-153). As a general rule, maximum efficiency of nucleic acid rehybridization occurs under reaction conditions involving moderately elevated temperatures (> 30°C) and the maximum rate of reaction between complementary strands occurs at a temperature about 25°C below Tm (J. MARMUR et al., J. Molec, Biology 3 (1961), 585-600). Thus, the device for achieving the present process involving temperature controlled hybridization and denaturation can be readily optimized to operate at maximal binding efficiency by chosing the appropriate temperatures according to the above relation.

A further object of temperature control is related to the "goodness-of-fit" between two strands of nucleic acid. By using a temperature gradient through the cell, an exact measurement of the Tm can be made when subjecting the hybridized molecule to de-annealing and monitoring kinetically. The Tm changes with goodness-of-fit become smaller as the fit becomes worse (R.B. WALLACE et al., Nucleic Acid Res. 6 (1979), 3543-3557). Therefore the device incorporating the technique of the present invention enables to accurately measure Tm, compare the observed Tm for a sample DNA with an expected Tm and thus accurately determine the actual goodness-of-fit of the DNA strands of interest. This is very important in detecting partial base-pairs homology which may exist with certain related microbial organisms, or in detecting single-point mutations related to some inherited diseases. Other techniques involving goodness-of-fit checking to which the present invention applies include for instance the followings: Ionic Strength variations (B.J. McArthy et al., Biochemical Journal 2 (1968), 37-48). Formamide concentration (B.C. Mc CONAUGHY et al., Biochemistry 8 (1969), 3289-3295). Other chaotropic agents such as sodium periodate (K. HAMAGUCHI et al., J.A.C.S. 84 (1962), 1329-1338); sodium perchlorate, sodium thiocyanate and potassium iodide (D.R. ROBINSON et al., J. Biological Chem. 241 (1966), 4030-4042), dextran sulfate (G.M. WAHL et al., Proc. of the Nat. Acad. Sc. USA 76 (1979), 3683-3687. Recently, significant hybridization rate increases have been demonstrated using a proportion of polyethylene glycol in the reaction buffer (R.M. AMASINO et al, Annals Biochem. 152 (1976), 304-307). The optimal conditions for hybridization buffers will also take the above into account.

In the reactions aimed at immobilizing nucleic acids (single strand probes or duplex structures) on waveguide surfaces (glass or plastics), three main embodiments are possible: a) immobilizing the probe, i.e. known polynucleotide sequences, on the waveguide and subsequently react the "probe" on the waveguide with an analyte of DNA to be determined. b) Immobilizing the hybridization product via a special binding sequence on the probe and c) immobilizing the mixture of DNA to be analyzed on the waveguide and react the latter with a solution of known "probe" DNA. Techniques (a) and (b) are particularly important in this invention.

For achieving immobilization of DNA on a substrate, the latter can be made nucleophilic, i.e. the substrate will thereafter bind with electrophilic nucleic acids centers (electron deficient sites, e.g. C=O of activated carboxylic esters, or phosphate esters). The substrate can also be made electrophilic (for instance charged positively) and attract nucleophilic nucleic acids (e.g. phosphate). This is the alternative illustrated on fig. 2 in which the duplex DNA will lie flat on the substrate surface after dye intercalation, although the probe is immobilized covalently at one point only (3' or 5' end). It should be noted that anchoring at only one single point enhances reactivity since the chain is able to freely wind and unwind in the solution. Then, orientation of the fluorescent complex parallel to the waveguide interface will enhance interaction of the fluorescent centers with the evanescent wave component and amplify the test sensitivity.

Attaching the probe only at one end required developing special chemistry.

A few techniques have been reported for linking non-modified DNA on cellulose or agarose. The following references are indicative:

M.S. Poonian et al.; Biochemistry 10 (1971) 424-27; P.T. Gilham Adv. Exp. Biol. Med. 42 (1974), 172-185; P. Venetianer at al.; P.N.A.S. USA 71 (1974) 3892-3895; D.J. Arndt-Jovin et al., Eur. J. Bioch, 54 (1975), 411-418; T.Y. Shih et al., Biochemistry 13 (1974), 3411-18; B.E. Noyes et al., Cell 5 (1975), 301-310; M.L. Goldberg et al., Methods in Enzymol. 68 (1979), 206-220; L.G. Moss et al., J. Biol. Chem. 256, (1981), 12655-58.

Usually low yields have been reported with solid support bearing either nucleophilic or electrophilic groups. Good results have been described using strong basic conditions with an electrophilic solid support (see MOSS above). But several conditions cannot be used with labile linkers on plates. Modification of DNA/RNA or at least activation of nucleic acids were also necessary to get good yields in mild conditions.

For instance, the waveguide surfaces were silanated with trihydroxy or trialkoxysilanes bearing a reactive group such as amino, SH, NCS, NCO, epoxy, azide, CHO, halide, carboxy, ester and the like. Silane compounds carrying 3-aminopropyl, 3-thiohydroxyalkyl, glycidoxypropyl can be used for silanation; then coupling the amine with an aryl or alkyl-diisothiocyanate will provide NCS terminated grafts. Polylysine can then be bound to the free NCS to increase the number of nucleophilic amino groups on the waveguide surface.

Probe DNA was synthesized by usual techniques, for instance methods suitable for making oligonucleotides. For this see for instance S.L. Beaucage et al., Tetrahedron Letters 22 (1981), 1859-1862; M.H. Caruthers, Chemical and Enzymatic Synthesis of Gene Fragments, Ed. H.G. Gasser & A. Lang (Verlag Chemie, Weinheim, FRG) (1981) pp. 71-79.

In one embodiment, an oligo-(dC)-3'-methylglucoside with about 25 nucleotides was immobilized, after oxidizing with periodate, on a glass plate previously silanated with an aminopropylsilane in a cyanoborohydride solution.

According to other embodiments, polyribonucleotides can be also immobilized using the same procedure. And polydeoxyribonucleotides can be modified enzymatically on their (3')-end by addition of a ribonucleotide using terminal deoxynucleotidyl transferase and ribonucleoside triphosphates (see R. ROYCHOUDHURY et al., Methods in Enzymology 65 (1980), 43-62). Then, as mentioned above, periodate oxidation and reductive condensation enable efficient immobilization of DNA on a silanated waveguide surface.

The synthesis of oligonucleotides with an amine group at the 3'- end was also performed. Thus, oligo (dC)-3')-methylglucoside (25-mer) oxidized with periodate as described earlier was purified chromatographically and coupled with 1,6-diaminohexane in tricine buffer. Then the product was reduced with cyanoborohydride, purified by H.P.L.C. and immobilized on an isothiocyanate grafted waveguide glass plate.

The modification of nucleic acid at their 5'-terminal was also achieved using techniques involving activation with imidazole or analogues under slightly acidic conditions. This technique was applied by analogy with the disclosure of B.C.F. CHU et al., Nucleic Acid Res. II (1983), 6513-29; P.N.A.S. USA 1982, 963-967. It was successfully applied to immobilize short synthetic DNA chains as well as long restriction fragments.

In short, oligonucleotides, e.g. dC (30) prepared by the solid phase method on a controlled pore glass support were phosphorylated with ATP and T4 polynucleotide kinase, the reaction course being monitored by means of a 1/10 aliquot labelled with $\gamma$-($32P$) ATP. The phosphorylated oligonucleotide was activated with $\beta$-imidazole in the presence of a carbodiimide, then it was linked to the surface of a wave guide plate silanated with an aminopropyl silane at pH 8.5.

Duplex DNA was also coated on a waveguide. For instance, phage $\lambda$ DNA was fragmented with appropriate endonucleases and the ethanol precipitated fragments were activated with imidazole and coupled to aminopropyl residues grafted on a waveguide plate by means of a carbodiimide. This construction was achieved to demonstrate operability of the invention using embodiment (b) mentioned before and also regarding optical fluorescence response upon addition of an intercalator dye. For performing the actual optical measurements according to the invention, the set-up illustrated on fig. 4 was mostly used. The waveguides used were planar glass or injection moulded plastic microscope slides. The angle of incidence was controlled by mirrors M1 and M2. The solution to be analyzed was pumped into the flow-cell 37 and fluorescent response generated at the interface was detected either at right angle (53) or at an output of the waveguide (40). Filter 52 blocked residual emission wavelength. The signal generated by detector 40 (or 53) was then processed in the electronic elements 41-45 and displayed according to usual means.

In addition to test sensitivity modulation by controlling the actual surface density of probe DNA on the waveguide surface, other sensitivity amplifying techniques were made available from prior art teachings in the field of DNA detection in solution.

For instance, in the present invention, it is obvious that the magnitude of the signal obtained by multiple internal reflectance photometry depends on the concentration of the fluorescence generator sites within the space available to the evanescent wave component. For a given intercalator dye, this response is a function of the total amount of duplex DNA within that space (assuming naturally that the linear density of intercalating sites remains constant).

However, this duplex DNA present may not necessarily be restricted to the hybrid involving the original probe DNA associated with complementary annealed test DNA. Means exist to supplement the early formed duplex structures, these means including, for instance, extending existing polynucleotide probe chains and hybridizing single-stranded DNA complementary to the extended sequence; another route would be adding double stranded preformed hybrids.

One approach is to supplement the reaction medium including probe hybridized material with DNA complementary to test DNA, i.e. to that portion of test DNA sequences not homologous with probe DNA. The result of this approach is to increase the amount of base pairs available for fluorescent site formation within the optically explored space at the waveguide interface.

In a variant, the supplemental DNA can be provided in prehybridized form which will further increase available chain length and fluorescence site concentration. Further amplification can still be provided by network formation by alternating complementary and non-complementary sequences.

Another approach is to provide extraneous fluorescent sites as prelabelled complementary DNA, labelling being done with fluorescing complexes such as FITC; thus, if fluorescing nucleotide substitutes are used for the synthesis of supplemental DNA that interacts as indicated, this will increase total fluorescence within the optical region of interest.

Regarding the second variant b) above (see page 21) where the probe is reacted with the analyte DNA in solution before the duplex is bound to the waveguide, the major advantage is increased kinetics of hybridisation. Here, the probe has its immobilisation sequence (for instance one moiety of a complex immuno-pair) attached to the non-hybridizing end and the waveguide is coated with the respective binding partner. Examples of several possible embodiments are given in the above references and include the following specific cases:

i): - Adding a polynucleotide to the (3') end of the probe and coating the waveguide with the complementary polynucleotide.

ii): - Coupling one (or more) moiety of an immunological binding pair to the probe and coating the waveguide with the immunological binding partner (e.g. biotin-probe and anti-biotin antibody on the surface).

iii): Coupling one (or more) moiety of a natural or modified binding pair to the probe and its respective partner to the waveguide (e.g. biotin-probe and avidin on the surface).

Working details are now provided in the experimental section which follows:


Experimental Part


1. Preparation of waveguide surface for linking DNA


a) Applying nucleophilic sites, e.g. grafting with γ-amino propylsilane:

Waveguides in the form of glass or plastic moulded microscope slides were scoured in cleaning solution (for instance glass was etched for 30 min at room-temperature in $F_3C$-COOH) and immersed under argon in a 2% by weight toluene solution of 3-aminopropyl-triethoxysilane and left there over night at 100° under argon. Then the plates were successively washed with methanol and water and dried under vacuum. The grafting density was approximately $10^{-10}$ to $10^{-9}$ moles/square cm but can be more.

The grafted plates were stored under argon before use. acetone solution.


a2) Grafting with γ-mercaptopropylsilane

The above procedure was used but replacing the amine silane by γ-thiohydroxypropyl-triethoxysilane. The grafting density was approximately as mentioned above.

b) Providing electrophilic sites, e.g. grafting with isothiocyanate

Plates obtained as under (a) above were dipped for 2 hrs in a 5% by weight dry DMF solution of 1,4-phenylenediisothiocyanate. Then the plates were washed in DMF, dried with ether and stored under argon.


c) Coating waveguide with polylysine to increase the amine density on the wave guide

A waveguide plate grafted with isothiocyanate groups as disclosed under (b) was reacted for 3 hrs in 0.1 M Hepes buffer (pH 7.6) with an excess of a $10^{-3}$M polylysine solution ($M_w \simeq 4000$) at room temperature. The plate was washed with methanol and water, it was dried and stored under argon before use. The coating density was approximately 50 picomole of lysine/$cm^2$ of the waveguide surface.

d) Providing positively charged groups on a waveguide

Plates as obtained under (a) or (c) above were quaternized with methybromide or methyl sulfate according to usual procedures. This quaterniza- tion reaction involved about 10 to 40% of the available amino groups. Alternatively, binding quaternary ammonium compounds on a waveguide surface can be brought about by treating the surface with an aqueous solution of a silane of formula $(MeO)_3Si-(CH_2)_3-NR_3$ where R is a lower alkyl, e.g. Me or Et. A silane of this kind is available commercially.

2. Modifications of nucleic acids for linking to the grafted waveguide

a) 3'-end modification of oligonucleotide with a terminal sugar or ribonucleoside - synthesis of CPG bearing an immobilized methylglucoside group

Methyl α-glucopyranoside (1.94 g, 10 mmoles) was etherified with a dimethoxytrityl protecting group (DMT) by usual means using the corresponding chloride (4.2 g, 11 mmoles) in pyridine (20 ml) and DMF (30 ml). The resulting Methyl 6-O-dimethoxytrityl-glucoside (3.3 g, 62%) was reacted in pyridine containing dimethylaminopyridine (1%) with succinic anhydride (1.8 g, 3 equivalents), and the product was purified by flash chromatography on silicagel (eluent CHCl3/MeOH 80/20 with 1% pyridine). After evaporation of the eluate, the acid was taken up with pyridine again and evaporated to eliminate the remaining methanol; yield 2.5 g. The succinylated sugar was dissolved in dioxane (12 ml) containing pyridine (1.5 ml), DCC (0.88 g, 4 moles) and p-nitrophenol (0.56 g, 4 mmoles), and this solution was shaken for two hours at room temperature. Dicyclohexylurea was removed by filtration and the solution used directly for coupling to controlled pore glass (CPG) beads [CPG/long chain alkyl amine (LCAA); pore diameter: 500 Angstroms; grade 24875 from Pierce Inc.] CPG (5 g) was quickly washed with a solution of diisopropylethylamine (5% in ether) and suspended in dry DMF (30 ml). The p-nitrophenylsuccinylated sugar derivative was added and the suspension was left at room temperature under gentle shaking. After washing with DMF remaining free amine and hydroxyl groups were capped with acetic anhydride (1 ml) in pyridine (7 ml) containing dimethylaminopyridine (1%). After washing (DMF) the CPG beads were resuspended in dry pyridine (10 ml) containing DCC (0.6 g) and p-nitrophenol (0.4 g) and gently shaken for six hours. Morpholine (0.4 ml) was then added and the suspension shaken for an additional 15 minutes.

After washing and drying the solid support was assayed for the trityl cation: 19 μmoles of sugar was immobilized per gram of CPG.

## Chemical synthesis of oligonucleotides bearing a (3')-modified end

CPG (53 mg; 1 µmole of sugar) was put onto the bottom of a 2.5 ml glass syringe fitted with a macroporous polyethylene filter, and the oligonucleotide was synthetized in this device using the phosphoramidite method disclosed by S.L. Beaucage et al. Tetrah. Letters 1982, 22, 1859.

The open syringe with CPG in it was put onto a test tube and rinsed with dichloracetic acid (2%) until (5')-end deprotection is complete. After washing sucessively with acetonitrile and DMF the syringe was reassembled, and the solid support was dried with three successive washings by sucking in dry acetonitrile through a septum using a needle fitted to the syringe. Then CPG was suspended in dry acetonitrile (1 ml) and the solvent was pushed back into a 1.5 ml plastic Eppendorf tube containing dry deoxycytosine nucleoside phosphoramidite (16 mg; 20 equivalents). This dissolved building block was then pushed back into another 1.5 ml plastic Eppendorf tube containing tetrazole (14 mg; 200 equivalents). Finally the dissolved activated phosphramidite was sucked into the syringe. And the syringe was put under rotation on a mechanical rotator for three minutes. After washing with 2,6-lutidine the unreacted species were capped with a solution (1 ml) of acetic anhydride (5%), lutidine (20%), dimethylaminopyridine (10%) in THF for two minutes.

Then the plugger of the syringe was removed and all remaining oxidation-, washing- and acid treatment steps were done with the open syringe put onto a needle inserted in a septum covering a vacuum Erlenmeyer flask.

Iodine solution (2 ml) containing iodine (1.7 g), THF (40 ml), 2,6-lutidine (20 ml) was added into the barrel. After 30 to 45 seconds CPG was washed with acetonitrile and dichlormethane. After a new treatment with dichloracetic acid (2%) the solid support was ready for a new addition of a new building block.

Oligonucleotides were made with methyl glucoside- and nucleosides-derivatized supports using the technique described. At each cycle a 20-fold excess of the desired nucleoside phosphoramidite was used, and the cycle was repeated the number of times necessary. It should be noted that these current synthetic steps can also be carried out with a gene machine.

Release and deprotection were done as described in M.H. Caruthers, Chemical and enzymatic synthesis of gene fragments, GASSER & LANG (Verlag Chemie, Weinheim, BRD; p.71-79 (1982) and in Oligonucleotide synthesis, a practical approach, M.J. GAIT Editor (IRL Press, Oxford, p. 35-81 (1984).

### (3')enzymatic modification of oligo-d(C)

Oligo d(C)-mer) was made starting with protected-d(C) derivatized CPG and purified by gel electrophoresis (20% urea gel) and HPLC on reversed phase C-8 column (25 cm) after complete deprotection.

Oligo d(C) (40 µg), 10x transferase buffer (20 µl), rCTP (2 nmoles) and terminal transferase (40 units) in a final volume of 200 µl were incubated for 6 hours at 37 degrees. After EDTA treatment (0.5M;20 µl) and extraction with phenol/chloroform as usual the resulting nucleic acid was desalted through a Sephadex G-50 column (Pharmacia Fine Chemicals) and detected by UV absorption (solvent :1 mM Tricine buffer).

### Periodate oxidation of oligo d(C)-(3')-methyl glucoside or oligo d(C)-(3')-oligo rC

Oligo d(C)-(3')-methyl glucoside (25-mer) (10 Gµug in 100 µl) or oligo d(C) (30-mer) cytidinated at the (3')-end (10 µg in 100 µl) was reacted with sodium metaperiodate (1% solution; 1 µl) at 4 degrees. After 30 minutes NaCl (4M; 100 µl), $Na_2HPO_4$ (0.5M; 100 µl) and glycerol (1% solution; 10 µl) were added. After an additional incubation at 4 degrees for 30 minutes the solution was lyophilysed and the residue desalted through a Sephadex G-50 column as described earlier. Finally the nucleic acid solution was adjusted to 1 ml.

### Immobilization of oligo d(C) on aminopropyl glass plate

An aminopropyl-derivatized glass plate was covered with the periodate oxidized oligonucleotide solution (500 µl; 3-5 µg) in Tricine buffer (pH 7.0). It was left for three hours with occasional shaking. Then cyanoborohydride (1% solution; 1 µl) was added, and the reaction was left for an additional two hours. After washing extensively with water the plate was kept under argon at 4 degrees until use.

The afore-mentioned technique was also used for preparing oligo d(A) coated waveguides.

### Immobilization of nucleic acid bearing nucleophilic amine at its (3')-end.

Oxidized oligo d(C)-(3')-methyl glucoside (25-mer) (about 10 µg) was desalted as usual, and half of the solution was reacted with 1,6-diaminohexane (1% solution; 1 µl) for two hours at room temperature in Tricine buffer (0.1M; pH 7.0). Then cyanoborohydride (1% solution; 1 µl) was added, and after an additional two hours at room temperature the resulting compound was purified by H.P.L.C. on a C-8 column in ammonium acetate buffer (0.1M; pH 5.5) Immobilization was done onto an isothiocyanate-derivatized glass plate in borate buffer (0.1M; pH 9.5) for two hours at room temperature.

### b) (5')-end modifications of nucleic acids

Direct condensation of (5')-phosphorylated nucleic acids in presence of soluble DCC and a "nucleophilic" solid support are usually poor. But through activation with imidazole or analogues at pH around 6 condensation has been shown to be more efficient and specific, as reported by Chu B.C.F. and Orgel L.E.; P.N.A.S 1985, 82, 963-67 and Chu B.C.F., Wahl G.M., Orgel L.E.; Nucleic Acids Research 1983, 11, 6513-29.

This procedure has been used to immobilized short synthetic DNA chains as well as long restriction fragments.

### Activation and immobilization of synthetic oligo d(C) (30-mer) at its (5')-end

Oligo d(C) (30-mer) (40 µg) was phosphorylated with T4 polynucleotide kinase (10 units) in kinase buffer at 37 degrees for two hours in presence of ATP.

Phosphorylation was followed in a 1/10 aliquot using γ (32P)-ATP. After work-up and lyophilysation the oligonucleotide was desalted through a Sephadex G-50 column.

About 4 µg was dissolved in 300 µl of imidazole. HCl buffer (0.1M; pH 6.1) containing N-Ethyl-N'-(3-dimethylaminopropyl)-carbodiimide hydrochloride (soluble DCC) (0.1M). And the solution was left for three hours at room temperature with occasional shaking. The activated nucleotide was purified by reversed phase HPLC using a triethyl ammonium acetate (0.1M, pH 7.0)/acetonitrile gradient. It was then reacted with a waveguide plate bearing amino-groups in 10 % lutidine/water solution at 60°, pH 8.5 for 4 minutes. Then the plate was washed extensively and kept at 4 degrees until use.

### c) Immobilization of single and double stranded DNA

λ DNA (3 µg in 50 µl) was treated with restriction enzyme Hpa II in low salt buffer for two hours at 37 degrees. After usual work-up, the DNA was ethanol precipitated and taken up in imidazole buffer (300 µl; pH 6.2) containing soluble DCC (0.1M) and NaCl (0.5M). The solution was left at room temperature for two hours with occasional shaking. Citrate buffer (0.2M; 300 µl; pH 8.4) containing NaCl (4M) was added to the activated aminopropyl glass plate that had been washed previously with diisopropylethylamine (5%) in ether. The activated DNA was added and mixed on the plate and left at 40 degrees for half an hour. After washing with NaCl (2M) the plate was left under argon at 4 degrees in a closed atmosphere.

Alternatively single stranded DNA was linked to the glass plate in the absence of NaCl by heating at 70 degrees for 4 minutes. A new solution of digested λ DNA was denatured by heating for 3 minutes at 100°C degres and added to the plate covered by NaCl solution (4M; 300 µl) and maintained at 80°C for 3 minutes. Then the solution was cooled slowly whereby hybridization occurred.

### d) Extension of the immobilized probe along hybridized nucleic acid by the fill-in technique using the four dNTP and DNA polymerase I (Klenov fragment)

A synthetic 45-mer of the non-coding strand of pBR-322 (nucleotide No 3871 to 3916) was immobilized on a waveguide as shown under section 2(b).

EcoR I digest of pBR322 was denatured for 3 min at 100 degrees in 200 µl buffer B solution (40 mM Tris.HCl pH 7.5 20 mM MgCl2. After a quick chilling 1M NaCl (200 µl) was added, and this single strauded DNA was hybridized to the probe coated on the waveguide (5 min at 80 degrees with slow cooling to room temperature (15')).

After washing with buffer containing NaCl (0.5M) and dithiothreitol (1mM) 40 µl NTP (3 mM of all four deoxyribonucleoside triphosphates) were added to 400 µl of buffer B containing NaCl (0.5M) and dithiothreitol (1nM) at room temperature, and then 20 units of Klenov large fragment were added.

e) Example of "sandwich hybridization" by using a second probe bearing an oligo dC- (3')-end and poly dG and poly dC to make a double stranded complex network

EcoR digest of plasmid pBR322 (1μg) was denatured for 5' at 100 degrees and was hybridized to a synthetic 45-mer attached at its (5') phosphate end to the waveguide (Nucleotide No 3871 to 3916 of the non coding strand). A second oligonucleotide (0.5 μg) whose primary structure is nucleotide No 70 to 100 of the non-coding strand of pBR322 was elongated at its (3') with dCTP (5mM) in cacodylate buffer (according to Meth. of Enzymology No 65, p. 435). After hybridization of the second probe to the immobilized nucleic acid poly dG and poly dC were added to make a double stranded complex network.

f) Example of "sandwich hybridization" (like the previous example), in which deoxycytidine of poly dC is replaced by N4-(6-Aminohexyl)deoxycytidine (dah dC), and was reacted with FITC

Poly(dC) (5A units; 0.3-0.4 mg) in 1 ml of freshly prepared solution of 1M $Na_2S_2O_5$/1M 1.6-diaminohexane/10mM hydroquinone (pH 7.3) was heated at 60 degrees for 4 days. Then the solution was dialyzed against 100mM borate buffer (pH 9.0) and passed through a Sephadex G-50 column. This modified polymer was reacted with fluorescein isothiocyanate in borate buffer (pH 9.0) to produce fluoresceinated poly(dah dC), which was used as in the previous example in a "sandwich hybridization" experiment with poly dG to make a complex network.

g) Example of a solution hybridization followed by immobilisation and (detection of the hybridised complex disclosed in next section.)

Briefly, a simple generic sequence, for example poly-dG is bound to the waveguide. A second probe element, a contiguous poly-dC of length similar to the poly-dG, carrying a sequence complementary to nucleotides, say 1-50, of the analyte sequence is prehybridized in solution with said analyte sequence; then the hybridized signal generating duplex hybridizes with the bound generic sequence.

Experimentally, as in e) above, the prepared oligonucleotide (0.5 μg) of 70 to 100 b.p. of the non-coding strand of pBR322 elongated at its (3') with dCTP was hybridised to heat denatured EcoR I digest of plasmid pBR322 (1 μg) for 5 min. The solution was then reacted with a waveguide coated with polydG to immobilise all the probe.

The sensitinaty of the above technique can be further amplified (in case the test sequence has, say, 200 bp) by adding, prior to hybridization with probe DNA, a third probe sequence element complementary to, say, nucleotides 55-200 of the test sequence, hybridization of this additional element being effected under optimal conditions using probe excess. The partial hybrid is then hybridized to the probe bound to the waveguide. Thus, the signal generating potential of the hybrid is therefore enhanced.

### 3. Experiments to measure DNA

The apparatus used has been described hereabove in connection with figs 4A, 4B and 4C. The waveguide 38 is a planar injection moulded plastic microscope slide. Light from a flash lamp 36 is directed through a monochromator 39 to select the specific wavelength of interest. The angle of incidence of the excitation light at the waveguide/liquid interface is controlled by using the two mirrors M1, M2. Light is coupled into and out of the waveguide using two quarter-round quartz prisms, 46, 47. Fluorescent light is detected either at right angles to the waveguide with a detector 54 or fluorescent light tunnelled back into the waveguide is detected at the waveguide output with a detector 40, including a photomultiplier tube positioned to detect fluorescent light which passes through the emission filter 52. The system is illustrated in the mode to collect tunnelled fluorescent light but is readily adjusted to measure fluorescence at right angles by repositioning the filter/detector as in fig. 4A. The signal is pre-amplified at 41, and passed to the microprocessor-controlled flash control/data acquisition system 12-45. The digitized signal is then stored on floppy discs 45, or printed as hard copy 44. Samples are introduced using a flow cell 37, and the volume in contact with the waveguide surface is defined by a rubber gasket 50. Samples are injected into the cell using an automatic pipette (e.g. Microlab P, Hamilton Industries, Bonaduz, CH) which reproducibly controls injection volumes and flow rates. In general, the probe nucleotides were coupled to the waveguide using the techniques detailed in Section 2 above. The grafted waveguide was then placed in the optical set-up and the flow-cell positioned on top as shown in fig. 4C. Then a solution of hybridization buffer (NaCl M) was placed in the cell and, after start-up, a base line was obtained on the display. Then the specific reaction was undertaken by adding a test solution to the cell and monitored by the change in fluorescence either at the waveguide output or underneath the waveguide.

Two basic experiments were carried out:

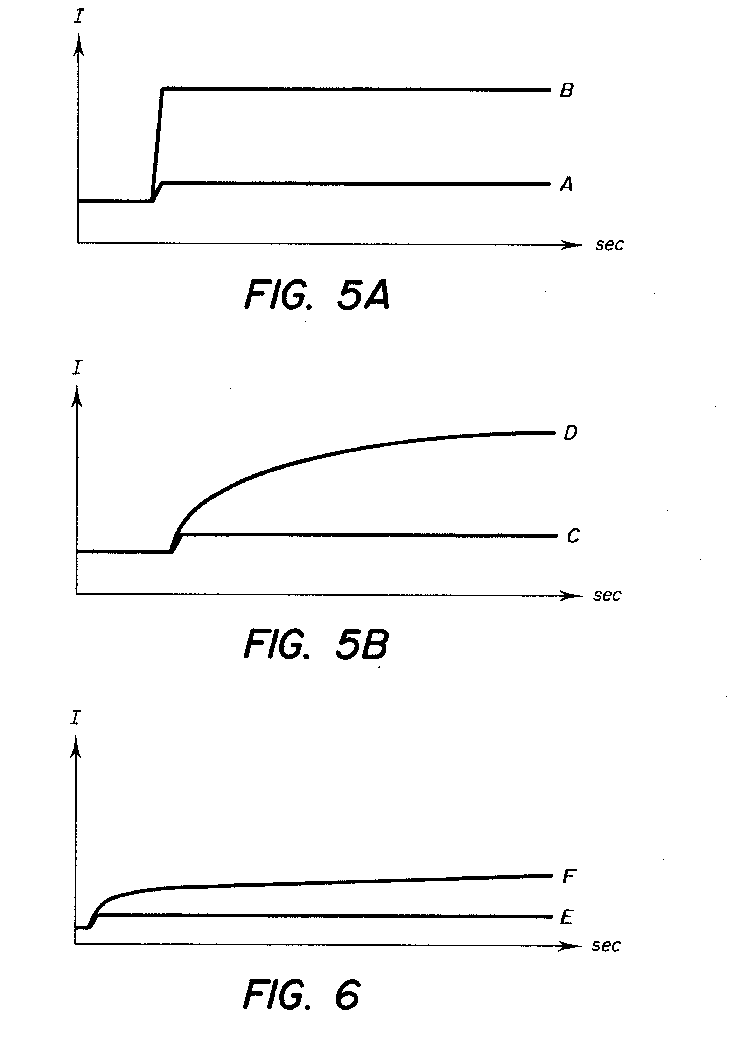

a) End-Point (Fig. 5A). This is actually a control experiment to show the feasibility of the test, i.e. build-up of a sufficiently strong signal upon addition of an intercalator dye to double-stranded DNA. In a first variant, waveguides prepared as under section 2 (c), i.e. coated with non-denatured λ DNA fragments were treated with various quantities of ethidium bromide in the same buffer (NaCl M; 100 µg of dye/ml stock solution). Useful responses were obtained with ethidium bromide in concentrations in the cell from 1 to 100 µg/ml. Naturally the response was also proportionnal to the density of the DNA on the waveguide.

Fig 5A illustrates the conditions where the DNA probe used had a density of approximately $10^{-3} - 10^{-1}$ µmole λDNA/$cm^2$ of the probe surface against a concentration of 60 µg/ml of ethidium bromide in the cell. In fig. 5A, the line B represents the increase in response resulting from the addition of the dye to the cell in the presence of the duplex DNA coated waveguide. Line A is the control obtained under identical conditions but no DNA on the waveguide. The slight jump in curve A is due to intrinsic solution fluorescence and the small fluorescence signal from non-intercalated dye.

In a second variant, single stranded DNA coated waveguides were used. The probe plates used were that obtained according to the procedure numbered 2 (c) of section 2 above followed by denaturation and comprised single stranded DNA derived from λ DNA. Then, a DNA solution containing denatured DNA was added. This solution was prepared by denaturing a mixture of DNA after treating with Hpa II endonuclease as indicated under section 2(c), first paragraph above. After addition of the single stranded DNA, hybridization was allowed to proceed for 20 min at room temperature after which ethidium bromide solution was added as in the first variant above. A response typically similar to that of fig. 5A, line B was obtained. For a given amount of ethidium bromide reagent, it was proportional to the complementary DNA in the analyte sample.

In a third variant, the effect of elongating the double helical polynucleotide after hybridisation, was tested.

For this, reference is made to section 2(d) above. In the procedure of this section, first paragraph, a pBR322 digest denaturate was hybridized with a synthetic polynucleotide probe. When ethidium bromide was added to the hybridization solution, a response curve of the kind illustrated by curve B of fig. 5A was obtained.

When a similarly prepared waveguide was contacted for 15 min at room temperature with the solution containing the elongated hybridized product disclosed in the second paragraph of section 2(d) above and then ethidium bromide in 0.5M aqueous NaCl was added, a response curve about twice the height of the first signal was obtained.

In a fourth variant, solution hybridisation and detection of the hybridised complex after formation, by constructing a probe oligonucleotide which contains an immobilisation sequence, was tested. Here the sequence was a polynucleotide of repeating known bases, but it can equally be any repeating polymer which will allow immobilisation of the complex to the surface via its complementary binder but which will not interfere with the hybridisation reaction with the probe (e.g. immunological binding pairs, lectins and sugars, other proteins and hapten binders). To carry out a solution hybridisation followed by immobilisation and detection of the hybridised complex, the procedure outlined under 2(g) was followed, then ethidium bromide in aqueous NaCl (0.5M) was added and the fluorescence measured. The data shows two response curves similar to curve B of fig. 5A but of different height. A first curve, the lower one, is the response obtained in the absence of the denatured target DNA pBR322, and the signal is derived from hybridization of only the annealing probe-polydC to the polydG affixed to the waveguide.

Another similar curve, a higher one, was obtained after hybridizing the probe to the target DNA and the carrying out the immobilizing reaction; this demonstrated the presence of the hybridized probe/pBR322 complex and proved that the probe did bind to the surface of the waveguide after hybridizing with its complementary DNA. Thus solution hybridization can be carried out and the hybridized product can be measured according to the method of the invention. Solution hybridization may have particular advantages in some cases in terms of speed to results.

b) Kinetic response.

The procedure was essentially the same as for the second variant of embodiment 3 (a) above with the difference that the ethidium bromide (50 µg/ml in the cell) was injected simultaneously with the complementary DNA sample. The results are shown in fig. 5B. Here the base line C has the same significance as line A of fig. 5A. Line D shows the fluorescence increase rate which is proportionnel to the rate of hybridization of the complementary DNA in the analyte with the DNA on the probe. The height of line D is proportionnal to the amount of DNA in said analyte. The above results show that dye intercalation proceeds about instantaneously relatively to the rate of hybridization.

The general experimental conditions are given below.

At all times the hybridisation buffer was 1 mol/L NaCl in distilled water. Hybridisations carried out before reaction with the dye were reacted at 100°C. Hybridisations effected in the cell were carried out at 22°C. The detailed effects of temperature on in situ monitoring of hybridisations have not been determined yet. The excitation wavelength was chosen at 300 nm. A narrow band-pass interference filter was placed over the P.M. tube to measure fluorescence at 600 nm (type 600S10-25, Oriel). The incident angle of excitation light at the interface was selected, as close to the critical angle as possible, at 66°. This was found to be very important in maximising the system sensitivity as at this angle there are the maximum number of reflections and therefore the maximum area of the surface is sensed, and the field strength at the surface is strongest at angles close to the critical angle therefore increasing the opportunity to monitor the surface reaction. The concentration of ethidium bromide (Fluka Chemicals) was varied but found to be useful between 1 and 100 µg/ml.

## c) Effect of excitation wavelength

To demonstrate the effect of wavelength of excitation on signal detection a waveguide with double stranded DNA affixed to the surface was placed in the optical bench; excitation lambda was scanned from 300 to 600nm with buffer in the cell and after reaction with ethidium bromide. This was repeated with a clean waveguide without DNA on the surface to act as a control. The results of the four wavelengths 300, 360, 500 and 550nm are given below in arbitrary units;

| lambda | 300 | 360 | 500 | 550 |
|---|---|---|---|---|
| +DNA background | 24 | 26 | 10 | 11 |
| after dye addition | 555 | 72 | 101 | 92 |
| ratio | 23.1 | 2.8 | 10.1 | 8.4 |
| -DNA background | 18 | 25 | 8 | 12 |
| after dye addition | 185 | 40 | 35 | 30 |
| ratio | 10.3 | 1.6 | 4.4 | 2.5 |

The above results illustrate that 300nm is the best lambda to use with this particular system. These results also demonstrate that the intercalated dye exhibits similar fluorescence characteristics when the DNA is affixed to a surface as when the DNA is free in solution or in a gel.

d) Kinetic Monitoring of the formation of double stranded DNA

A plate waveguide with poly-dA affixed thereto was used (see preparation under section 2 (a)). Then, the waveguide was affixed into the flow cell and a base-line signal obtained with buffer in the cell. Both ethidium bromide and .25U/ml of poly-dT were mixed and injected into the flow cell at the same time and the reaction monitored. The results are given in figure 6, line F. A control experiment is also shown where only the ethidium bromide was injected into the cell (line E). These results demonstrate that the binding between complementary strands of DNA can be monitored as it occurs using the invention. Furthermore, the monitoring reaction is effectively immediate and only specific intercalation between double stranded DNA is detected. This again will be very useful for producing an analyser for carrying out DNA probe analysis for clinical and diagnostic use. Similar data were found with poly-dC and poly-dG but the signals were

0245206

stronger. A further test was carried out to illustrate the general utility of using the "sandwich" type hybridisation combined with forming a double stranded complex netword with polydC: polydG to give many intercalation sites. The procedure detailed in section 2(e) was followed and hybridation was carried out in the presence of ethidium bromide. The rate was measured by fluorescence as above and curves similar to that illustrated in fig. 6 were obtained. The first curve (the lower one) represents the signal obtained before the final step of complex formation. A second curve (similar to F in fig. 6) was obtained by mixing the two polynucleotides (polydG, and polydC) with the ethidium bromide and monitoring the reaction kinetically. These data illustrate the increased sensitivity of this approach over noncomplex formation.

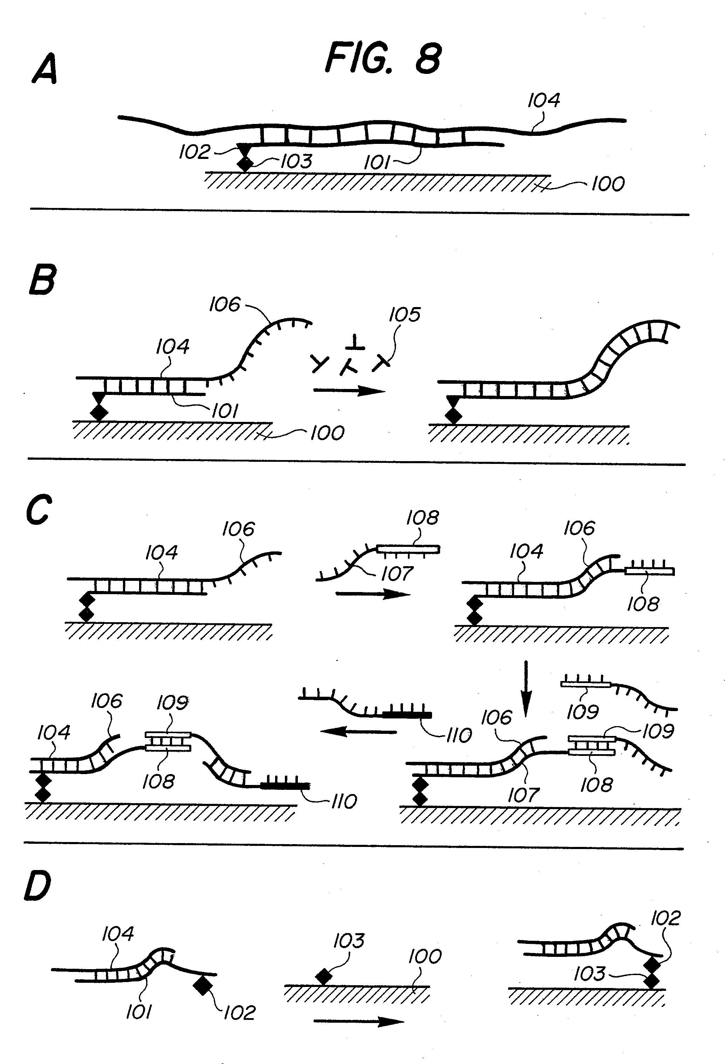

Fig. 8 illustrates, by a series of diagrams (A, B, C and D) some of the main analytical techniques embodied by the present invention. The purpose of these diagrams is to provide a short summary of the various methods disclosed in this specification.

Diagram A shows a waveguide 100 on which a probe polynucleotide 101 has been atteched by a linker 102 to a binding site 103 of the waveguide. The diagram also shows a segment of analyte polynucleotide 104 of which the center section is complementary to the probe and which is hybridized to the probe.

Diagram B shows the case of a piece of DNA 104 hybridized with a probe 101 attached to a waveguide 100 the single ended strand 106 of which has been filled by the complementary bases 105 for expansion (see section 2(d)).

Diagram C illustrates the progressive supplementation of DNA 107 complementary to free section 106 of test DNA 104, this supplemental DNA 107 carrying a further sequence 108 to be even further supplemented afterwards by DNA 109 and 110. These supplemental sequences can be synthetic homopolynucleotides (poly-dA, poly-dT, poly-dC, poly-dG). This illustrates the "sandwich hybridization procedure of section 2(e). This elongation increases the number of fluorescent sites by unit probe and increases sensitivity. It should be understood that the sequences defined by numerals 109 and 110, although represented as heterogeneous, can consist of the same or different nucleotides, the provison being that a portion of 109 is complementary to 108 and at least part of 110 is complementary to at least a part of 109. Thus, as an example, 109 could by all poly-dG and 110 could be all poly-dC.

0245206

It is also important to note that within the meaning of the embodiment illustrated by diagram C, the probe DNA can consist of a piece of DNA to be attached to the waveguide by hybridization with a complementary DNA already bonded to the waveguide by a link 102-103.

Diagram D illustrates solution hybridization of probe with test DNA 104 and subsequent attachment of the hybrid to waveguide 100 via linking members 102 and 103.

It should also be noted that the measuring technique of the invention applies as well to the monitoring of fluorescence variation (i.e. when hybridization occurs at the surface of the waveguide or when the duplex resulting from solution hybridization attaches to the waveguide) and to end-point determinations (i.e. the measurement of fluorescence at an output of the waveguide after the reaction is complete or after the hybridized product of interest has attached to the waveguide). In this case, the output signal can be either measured when the waveguide is still in contact with the analyte sample solution or after separating the waveguide from the solution. The advantage of effecting this separation before measuring the signal is to decrease or eliminate the solution back-ground noise.

C L A I M S

1. A method for determining nucleic acids (a) featuring one or more polynucleotide sequences of interest present in a liquid analyte sample mixture of polynucleotides, this method including:

i) contacting said sample in a reaction medium with a polynucleotide probe (b) featuring polynucleotide conterparts to said sequence of interest in the presence of a solid support having binding affinity for said probe, whereby hybridization between (a) and (b) occurs with the formation of a two stranded duplex structure,

ii) adding a label compound designed to specifically label said duplex, e.g. to intercalate between the strands of said duplex, thus providing a fluorescent complex signal generator indicating the formation of said duplex,

characterized in that said solid support is a waveguide to which said probe is bound before or after step i) and carrying an internally reflected wave signal which interacts with said bound complex at the surface thereof and produces a fluorescence response which is measured at an output of said waveguide thus providing data representative of said determination.

2. The method of claim 1, in which the probe is linked to the waveguide before step i, hybridization occurs at the surface of the waveguide and the fluorescence increase is representative of the rate of said hybridization i.e. of the amount of analyte nucleic acid in the sample.

3. The method of claim 1, in which step i) is started before the probe links to the waveguide whereby the fluorescence increase with time is indicative of the rate of binding of the duplex structure to the waveguide surface and the total end-point fluorescence relates to the amount of analyte nucleic acid in the sample.

4. The method of claim 1 where said waveguide is a rectilinear transparent solid body, in which a signal output of said waveguide is collected laterally at right angles to said waveguide or axially at an end of said waveguide or at both.

5. The method of claim 1, in which the waveguide is an optical rod or fiber or a plate forming a wall of an analytical reaction test cuvette.

6. The method of claim 5, in which an output signal of said waveguide is collected laterally at right angles to said interface the emitted fluorescent light being picked-up from a side of the waveguide opposite to said interface.

7. The method of claim 1, in which said interaction of the reflected wave signal and said fluorescent complex occurs mainly at a fraction of a wavelength away from the waveguide/analyte interface which enables to clearly distinguish the fluorescence produced by said complex linked to the waveguide from that occurring within the bulk of the analyte.

8. The method of claim 2, in which the nucleic acid probe is covalently attached to the waveguide by a terminal nucleotide, all or most bases of said nucleic acid being available for hybridization.

9. The method of claim 8, in which the probe polynucleotides aligns in parallel relationship with the waveguide surface, this orientation resulting from the presence on the waveguide surface of sites having electrostatic affinity for the phosphate moieties of said probe polynucleotides.

10. The method of claim 9, in which said sites result from the presence on the waveguide surface of positively charged groups, e.g. quaternary ammonium groups.

11. The method of claim 8, in which said covalent attachment is provided by first grafting on the waveguide trialkoxysilanes bearing reactive groups such as $-NH_2$, $-OH$, $-SH$, glycidoxy, $-NCO$ and the like and thereafter using these groups or derivatives thereof as linkers for the probe polynucleotides.

12. The method of claim 8, in which the nucleic acid probe consists of oligonucleotides in the range of about 10 to 105 nucleotides.

13. The method of claim 8, in which said covalent attachment is provided by reacting the probe nucleic acids with reactive groups previously incorporated to the waveguide substance.

14. The method of claim 1, wherein the waveguide is part or whole of a moulded disposable plastic cuvette.

15. The method of claim 1, wherein the waveguide is moulded from high optical quality plastics such as polycarbonate or polymethyl-methacrylate

16. The method of claim 1, wherein monitoring of the fluorescence signal is effected by either measuring said fluorescence at equilibrium (end-point determination) or by measuring the rate of change of the fluorescence as the reaction proceeds, or both.

17. The method of claim 1, in which the reaction is effected under controlled temperature conditions.

18. The method of claim 17, in which the temperature is varied continuously, linearly or according to a step-wise gradient.

19. The method of claim 18, in which the measured results are computed to provide the mean thermal denaturation temperature Tm of the hybridized product.

20. The method of claim 1, in which one or several chaotropic agents or polymers selected from dextran sulfate polymers, polyethylene glycol, sodium iodide and perchlorate salts are added to the reaction medium.

21. A kit for implementing the method of claim 1 comprising the following components:

- a sampling device for biological cell materials;

- a sample preparation device for extracting, deproteinising and separating the strands of the selected nucleic acid sample from said cell material;

- a cuvette-waveguide incorporating a nucleic acid probe;

- a means for introducing the prepared sample and an intercalator dye into the cuvette-waveguide;

- an optical device for illuminating the waveguide with a totally reflected signal and to time and monitor the fluorescence generated at the waveguide surface upon hybridization of the sample nucleic acid with the probe;

- a signal processing device and a display to electronically process the monitored fluorescence and display legible results representative of the said hybridization reaction.

22. The kit of claim 21, in which the cuvette-waveguide is either a disposable cheap molded cuvette or a reusable flow-cell.

23. The method of claim 1, including amplification of the fluorescence signal of interest by increasing the available fluorescent site concentration in the space optically probed by the excitation signal.

24. The method of claim 23, whereby said concentration increase is provided by extending the length of the duplex structure resulting from hybridization at the probe surface.

25. The method of claim 24, wherein said length increase is brought about by adding further nucleic acid homologous to the as yet not hybridized sequences in the bound test nucleic acid, whereby further annealing occurs with consecutive further dye intercalator sites provision.

26. The method of claim 1, in which a 5'-phosphate-linked nucleic acid probe is elongated along the hybridized strain using the four deoxynucleoside triphosphate and a DNA polymerase or a reverse transcriptase (fill-in technique), or both.

27. The method of claim 1, in which the probe binds to the waveguide by hybridization with complementary DNA linked to said waveguide or coated thereon.

28. The method of claim 1, in which the probe is linked to one partner of a binding pair and another partner of said pair is linked to the waveguide surface, whereby probe attachment to the waveguide proceeds via formation of the binding pair.

29. The method of claim 28, in which said one first partner is selected from antigens, haptens, glycosides, sugars, biotin, avidin and other binding proteins, the other partner being complementary to said first partner.

30. The method of claim 1, wherein the waveguide is a hollow tube and the probe is immobilized on the inner or outer surface thereof or on both.

31. The method of claim 1, in which said measuring is effected either during hybridization by monitoring fluorescence or, after hybridization is complete, by fluorescence end-point determination.

32. The method of claim 31 comprising fluorescence end-point determination, in which the measurement is effected either in the presence or in the absence of the liquid analyte solution.

0245206

FIG. 1

FIG. 2

FIG. 3A

FIG. 3B

FIG. 4A

FIG. 4B

FIG. 4C

FIG. 4D

0245206

I

B

A

sec

## FIG. 5A

I

D

C

sec

## FIG. 5B

I

F

E

sec

## FIG. 6

FIG. 7

FIG. 8

European Patent
Office

EUROPEAN SEARCH REPORT

0245206
Application number

EP 87 81 0274

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | EP-A-0 184 600 (BATTELLE MEMORIAL INSTITUTE) <br><br> * Whole document * | 1-7,10 ,11,13 -23,28 -32 | C 12 Q 1/68 <br> G 01 N 21/75 <br> G 01 N 33/543 <br> G 01 N 21/64 <br> G 01 N 33/545 |
| Y | EP-A-0 075 353 (BATTELLE MEMORIAL INSTITUTE) <br> * Whole document * | 1-32 | |
| Y | EP-A-0 128 723 (M.J. BLOCK) <br> * Whole document * | 1-32 | |
| Y | EP-A-0 175 585 (CORNING GLASS WORKS) <br> * Whole document * | 1-32 | |
| Y | EP-A-0 117 440 (ENZO BIOCHEM, INC.) <br> * Whole document * | 1-32 | TECHNICAL FIELDS SEARCHED (Int. Cl.4) <br><br> G 01 N <br> C 12 Q |
| D,A | EP-A-0 131 830 (MOLECULAR DIAGNOSTICS, INC.) <br><br> * Abstract; pages 1-3; page 8, line 19 - page 9, line 23; page 12, line 23 - page 14 * | 1-3,26 -29,31 ,32 | |

The present search report has been drawn up for all claims

| Place of search | Date of completion of the search | Examiner |
|---|---|---|
| THE HAGUE | 24-07-1987 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82